# EUROPÄISCHE PATENTSCHRIFT

(11) **EP 0 814 776 B1**
(45) Veröffentlichungstag und Bekanntmachung des Hinweises auf die Patenterteilung: **04.07.2001**
(21) Anmeldenummer: 96905579.7
(22) Anmeldetag: 14.03.1996
(51) Int. Cl.: A61K 9/06, A61K 9/16

(54) **TOPISCH APPLIZIERBARE PHARMAZEUTISCHE ZUSAMMENSETZUNG, VERFAHREN ZU IHRER HERSTELLUNG UND VERWENDUNG DER ZUSAMMENSETZUNG**
TOPICALLY APPLIED PHARMACEUTICAL COMPOSITION, METHOD OF PREPARING IT AND ITS USE
COMPOSITION PHARMACEUTIQUE D'APPLICATION TOPIQUE, SON PROCEDE DE PRODUCTION ET SON UTILISATION

(30) Priorität: 17.03.1995 AT 475955
(43) Veröffentlichungstag der Anmeldung: 07.01.1998
(73) Patentinhaber: Gebro Pharma GmbH, 6391 Fieberbrunn (AT)
(72) Erfinder: EISENREICH, Volker, A-6391 Fieberbrunn (AT); HANTICH, Gerhard, A-6370 Kitzbühel (AT); HESSE, Ernst, A-6391 Fieberbrunn (AT); MÖLLER, Torsten, D-83352 Altenmarkt an der Alz (DE)
(74) Vertreter: Wildhack, Helmut, Dipl.-Ing. Dr.
(86) Internationale Anmeldenummer: AT9600048
(87) Internationale Veröffentlichungsnummer: WO9629056

(56) Entgegenhaltungen:
- EP-A- 0 376 852

## Beschreibung

Die Erfindung bezieht sich auf eine topisch applizierbare pharmazeutische Zusammensetzung, welche enthält:
Zumindest ein flüssiges Lipid,
zumindest einen pharmazeutischen Wirkstoff, der im zumindest einem der flüssigen Lipide löslich ist und von der Haut resorbiert wird,
und ein wasserhältiges Gel, das den Hauptbestandteil der Zusammensetzung bildet, wobei zumindest ein Lipid und zumindest ein Wirkstoff in das Gel eingearbeitet sind und die Zusammensetzung im wesentlichen frei ist von Emulgatoren und festen Bestandteilen, mit Ausnahme zur Bildung des wasserhältigen Gels nötiger Geliermittel.

Unter einem wasserhältigen Gel wird hiebei ein formbeständiges, halbfestes Hydrogel verstanden, das durch Verarbeitung von einem oder mehreren Gelbildnem (oft als Verdickungsmittel bezeichnet) mit Wasser erhalten wird.

Eine derartige pharmazeutische Zusammensetzung ist bekannt (DE-A 3,336.047).

Die konventionellen halbfesten Zubereitungsformen von topisch applizierbaren pharmazeutischen Darreichungsformen umfassen im wesentlichen Hydrogele bzw. Lipogele, Emulsionen, Salben und Liposomenzubereitungen. Diese üblichen Zubereitungen sind zumeist mit Nachteilen verschiedenster Art behaftet und zwar:

Hydrogele wirken auf die Haut oft austrocknend, was bedingt ist durch den zumeist hohen Anteil an leichtflüchtigen organischen Lösungsmitteln, insbesondere Alkoholen.

Emulsionen enthalten ein Allergiepotential durch den Gehalt an Emulgatoren, so daß Irritationen der Haut hervorgerufen werden können.

Salben bzw. Lipogele weisen zumeist zu fette Grundlagen auf, die nur langsam in die Haut einziehen und oft durch Verfettung der Umgebung (Kleidung usw.) störend wirken.

Liposomenzubereitungen sind durch einen relativ geringen Beladungsgrad der Liposomen limitiert.

Anderseits ist es aber erwünscht, zahlreiche Wirkstoffe topisch zu applizieren. Dies gilt z.B. für Antirheumatika, Lokalanaesthetika, Antiallergika, durchblutungsfördernde Substanzen usw. Aus den oben erwähnten Gründen stellt es sich jedoch als schwierig dar, den Wirkstoff in eine Trägersubstanz einzuarbeiten, welche zugleich günstige pharmazeutische wie kosmetische Eigenschaften besitzt. So muß z.B. die Freisetzung des Wirkstoffes aus der Arzneiform gewährleistet sein (zumeist ist die Freisetzung auch noch rasch gewünscht), aber auch die Stabilität des Wirkstoffes und der Arzneiform selbst. Weitere Forderungen sind angenehme Applikation, schnelles Einziehen in die Haut und Vermeidung von Hautirritationen.

Die geschilderten Schwierigkeiten stellen sich in hohem Maße z.B. für das nichtsteroidale Antirheumatikum lbuprofen, da dieser Wirkstoff eine starke Tendenz zur Rekristallisation auch aus organischen Medien aufweist. Daher wurde bereits vorgeschlagen, diesen Wirkstoff in eine geeignete Matrix einzuarbeiten. Hiezu ist es bekannt, z.B. Benzylalkohol (GB-A 2 236 250) oder Menthol (WO 91/04733) einzusetzen. Die so erhaltenen Arzneiformen konnten jedoch ebenfalls aus den oben angeführten Gründen nicht völlig befriedigen. Ähnliches gilt für die eingangs erwähnte topisch applizierbare pharmazeutische Zusammensetzung (DE-A 3 336 047), welche ebenfalls als Wirkstoff u.a. Ibuprofen einsetzt, wobei der Wirkstoff in Kombination mit einem wasserlöslichen, flüchtigen Niederalkanol, Wasser, einem gegebenenfalls selbstemulgierenden Lipid und einem Gelgerüstbildner eingesetzt wird.

Es besteht daher das Bedürfnis, eine topisch applizierbare pharmazeutische Zusammensetzung der eingangs erwähnten Art zu schaffen, welche die geschilderten Nachteile vermeidet und universell einsetzbar ist, so daß je nach Bedarf eine effektive Therapie unter Vermeidung hoher Blutspiegel möglich ist. Insbesondere soll gewährleistet sein, daß der pharmazeutische Wirkstoff zwar aus der Arzneiform nach deren Applikation leicht freigesetzt wird, so daß er von der Haut rasch aufgenommen (resorbiert) werden kann, anderseits aber in der Arzneiform stabil und frei von Rekristallisation gehalten ist. Weitere Anforderungen sind, wie bereits erwähnt, daß die Zusammensetzung auf die Haut nicht austrocknend wirken und Hautirritationen vermeiden soll. Die Erfindung löst diese Aufgabe dadurch, daß alle Wirkstoffe in gelöster Form vorliegen, wobei jedoch zumindest ein Wirkstoff im flüssigen Lipid in gelöster Form vorliegt und daß dieses den Wirkstoff gelöst enthaltende Lipid als solches als innere Phase in das wasserhältige Gel als äußere Phase eingearbeitet ist, und die Zusammensetzung im wesentlichen frei ist von oberflächenaktiven Substanzen. Eine solche Zusammensetzung kann als "Dispersionsgel" bezeichnet werden. Sie stellt ein Zweiphasensystem dar, welches eine in einer wässerigen Gelphase verteilte Lipidphase aufweist. In dieser Lipidphase sind ein oder mehrere Wirkstoffe gelöst, was günstige Penetrationseigenschaften für den Wirkstoff ergibt. Vorteilhaft ist hiebei auch, daß wesentlich höhere Wirkstoffkonzentrationen am Wirkort erzielbar sind, als dies bei konventionellen dermalen Darreichungsformen vorliegt. Dies läßt sich dadurch erklären, daß - im Vergleich zu z.B. Emulsionen oder Salben - die erfindungsgemäße Zusammensetzung mit einem viel geringeren Fettanteil auskommt. Bei gleicher Wirkstoffkonzentration (die Gesamtzubereitung betreffend) ist bei der erfindungsgemäßen Zusammensetzung nach erfolgter Applikation auf der Haut und Verdunsten der äußeren wässerigen Phase der Wirkstoff wesentlich konzentrierter vorhanden als bei konventionellen Zweiphasensystemen, und er steht entsprechend dem 1. Fick'schen Diffusionsgesetz in größerem Maße zur Resorption zur Verfügung. Beispielsweise einsetzbare lipidlösliche Wirkstoffe sind Prednisolon, Fluocortolon, Triamcinolon, Hydrocortison, Fusidinsäure, Clotrimazol, Ciclopiroxolamin, Tolnaftat, Amphotericin B, Dithranol, Vitamin A, Vitamin E, Benzoylperoxid, Hexetidin, Estradiol, Bufexamac, Diclofenac, Ketoprofen, Piroxicam, Indometacin, Flufenaminsäure, Felbinac, Hydroxyethylsalicylat, Etofenamat, Naproxen, Polidocanol, Nicotinsäurebenzylester oder Ethylenglycolmonosalicylat, vor allem aber das nichtsteroidale Antirheumatikum Ibuprofen, insbesondere S(+)-lbuprofen, welches auch in handelsüblicher Pharmareinheit, also mit einem geringen Anteil an R(-)-lbuprofen eingesetzt werden kann. Letzterer Wirkstoff, aber auch andere bei der erfindungsgemäßen pharmazeutischen Zusammensetzung einsetzbare Wirkstoffe haben den Nachteil einer schlechten Löslichkeit sowie eine starke Tendenz zur Rekristallisation, auch aus zahlreichen organischen Medien. Es hat sich nun in überraschender Weise gezeigt, daß solche heikle Wirkstoffe, insbesondere S(+)-lbuprofen, welches im Vergleich zu racemischem Ibuprofen eine doppelt so starke analgetische bzw. antiphlogistische Wirkung hat, mit Lipiden Lösungen von solcher Stabilität bildet, daß im in die Hydrogelphase eingearbeiteten Zustand sogar im Vergleich zu herkömmlichen Emulsionen wesentlich größere Tröpfchengrößen der inneren Phase (d.i. die den Wirkstoff enthaltende Phase) tolerierbar sind, ohne daß Rekristallisationserscheinungen auftreten. Die Größe solcher dispergierter Tröpfchen des den Wirkstoff bzw. die Wirkstoffe enthaltenden flüssigen Lipids beträgt bis zu 60 um, wogegen die übliche Größe von Emulsionströpfchen in dermalen Zubereitungen im Nanometerbereich liegt.

Zudem ermöglicht es die Erfindung in einfacher Weise, den Wirkstoff bzw. die Wirkstoffe in der Lipidphase in hochkonzentrierter Form, vorzugsweise in übersättigter Lösung, einzubringen.

Dadurch, daß die erfindungsgemäße pharmazeutische Zusammensetzung im wesentlichen frei ist von Emulgatoren, werden deren nachteilige Einflüsse (Allergiepotential) mit Sicherheit vermieden. Analoges gilt für die Freiheit von oberflächenaktiven Substanzen und auch für die Freiheit von festen Bestandteilen, denn alle Wirkstoffe liegen in gelöster Form vor und das Lipid oder die Lipide ist (sind) flüssig. Das die äußere Phase bildende wasserhältige Gel ist nur halbfest und enthält mit Ausnahme der festen Verdickungsmittel keine festen Bestandteile.

Dadurch, daß das den Wirkstoff gelöst enthaltende Lipid als solches, d.h. ohne Veränderung seines Charakters, etwa durch Formulierung einer Emulsion durch Zugabe von Wasser (WO 93/18752), in das wasserhältige Gel eingearbeitet ist, werden auf Veränderungen des Lipids entfallende Arbeitsschritte und damit verbundene Kosten eingespart.

Gemäß einer bevorzugten Ausführungsform der Erfindung ist die pharmazeutische Zusammensetzung auch im wesentlichen frei von flüchtigen organischen Lösungsmitteln und bzw. oder im wesentlichen frei von flüchtigen niederen Alkanolen und bzw. oder frei von mit Wasser mischbaren Cosolventien, wie z.B. Propylenglycol, Glycerin oder niedermolekulare flüssige Polyole, Polyethylenglycol..

Der Ausdruck "im wesentlichen" soll bedeuten, daß geringe Gehalte der erwähnten Zusätze, z.B. Alkohol, tolerabel sind, solange sie die erwähnten wesentlichen galenischen Vorteile der erfindungsgemäßen pharmazeutischen Zusammensetzung nicht beeinträchtigen. Durch die Freiheit an den erwähnten Zusatzsubstanzen bietet sich die Möglichkeit für die Herstellung von hypoallergenen Zubereitungen und weiters kommen die eingangs erwähnten günstigen Eigenschaften am besten zur Wirkung. Trotz des Verzichtes auf flüchtige organische Lösungsmittel tritt kein unangenehmes Gefühl an der Haut und auch keine Überfettung derselben auf, offenbar bedingt durch die Eigenschaften des Gels, welches den im Lipid genannten Wirkstoffanteil enthält. Günstig ist im Rahmen der Erfindung ein hoher Wasseranteil der Zubereitung, der bis 90 Gew.-% (bezogen auf die Gesamtmenge) betragen kann. Trotz dieses hohen Wassergehaltes zeigt die erfindungsgemäße Zusammensetzung rückfettende Eigenschaften, d.h., das Gel wird von der Haut schnell aufgenommen und verursacht eine angenehmes Gefühl beim Patienten. Der pH-Wert der Zusammensetzung kann ohne Schwierigkeiten im physiologischen Bereich, d.h. zwischen 5 und 6 liegen.

Selbst bei völliger Emulgatorfreiheit und Freiheit an oberflächenaktiven Substanzen zeigt sich in überraschender Weise, daß das "disperse" System, welches das den Wirkstoff enthaltende Lipid mit dem Hydrogel bildet, stabil in jeder Beziehung ist.

Für die flüssige Lipidphase eignen sich im Rahmen der Erfindung zahlreiche natürliche und künstliche Fette bzw. Öle, insbesondere Ricinusöl und bzw. oder Mandelöl und bzw. oder Sesamöl und bzw. oder mittelkettige Triglyceride und bzw. oder Mischungen dieser Substanzen.

Zur Herstellung eines alkoholfreien Gels können zahlreiche bekannte Gelbildner eingesetzt werden, wie Hydroxyethylzellulose und bzw. oder Hydroxypropylzellulose und bzw. oder Polymere der Acrylsäure, wie sie z.B. unter dem Handelsnamen "Carbopol" (reg.Warenzeichen) bekannt sind.

Wie bereits erwähnt, ist es günstig, wenn der Wasseranteil der Zusammensetzung hoch, etwa bis 90 Gew.-% ist. Trotz des vorliegenden Überschusses am Nichtlösemittel Wasser kristallisiert der Wirkstoff im Gel nicht aus.

Im Rahmen der erfindungsgemäßen Zusammensetzung besteht auch die Möglichkeit, zusätzlich zu dem im Lipid gelösten Wirkstoff bzw. den dort gelösten Wirkstoffen zumindest einen Wirkstoff einzusetzen, der nicht in im Lipid gelöster Form im Gel vorliegt, sondern z.B. in in der wässerigen Gelphase gelöster Form. Dies ergibt neben dem Einsatz fettlöslicher Arzneistoffe die Möglichkeit des zusätzlichen Einsatzes wasserlöslicher Wirkstoffe in die äußere Gelphase. Auch für diese zusätzlichen Wirkstoffe gilt, daß die hautfreundlichen, rückfettenden, schnell einziehenden und kosmetisch angenehmen Eigenschaften des Dispersionsgels genutzt werden. Beispiele für derartige wasserlösliche Wirkstoffe sind Gentamycin, Neomycin, Bacitracin, Clindamycin, Erythromycin, Aciclovir, Vidarabin, Dexpanthenol, Allantoin oder Hirudin. Selbstverständlich können mehrere Arzneistoffe mit unterschiedlichen Lösungseigenschaften in der öligen Phase eingebracht sein und - wie erwähnt - auch zusätzlich in der wässerigen Phase.

Das erfindungsgemäße Verfahren zur Herstellung einer erfindungsgemäßen topisch applizierbaren pharmazeutischen Zusammensetzung der oben beschriebenen Art kennzeichnet sich dadurch, daß zumindest ein von der Haut resorbierbarer Wirkstoff in einem Lipid, vorzugsweise konzentriert, gelöst wird und daß sodann diese Lösung als innere Phase in ein Hydrogel als äußere Phase eingearbeitet wird, wobei das Hydrogel den Hauptbestandteil der Zusammensetzung bildet. Diese Arbeitsvorgänge bereiten im Prinzip keine Schwierigkeiten und können mit üblichen Apparaten ausgeführt werden. Zweckmäßig erfolgt der Lösevorgang unter Erwärmung, die abgekühlte Lösung wird anschließend in das Hydrogel eingearbeitet. Die Erwärmung braucht nur mäßig zu sein, um die Lösung des Wirkstoffes bzw. der Wirkstoffe im Lipid bzw. in der Lipidkombination zu begünstigen, sie soll nicht zu hoch sein, um thermische Schädigungen insbesondere der Wirkstoffe zu vermeiden.

Die Einarbeitung der Lösung in das Gel erfolgt durch Mischen bis zur Erreichung des gewünschten Dispersionsgrades, zweckmäßig mittels konventioneller Salbenkessel bzw. Salbenanlagen (z.B. solche der Firmen Fryma,Brogli, Suter). Der Dispersionsgrad bzw. die Größe der wirkstoffhaltigen Fett- bzw. Ölteilchen wird entscheidend durch die Wahl des Dispergierungswerkzeuges bestimmt. Hochtourige Werkzeuge (Homogenisatoren) erbringen Partikelgrößen bis 15 µm, während einfache Mischer (z.B. Schaufelmischer) Tröpfchengrößen bis 60 µm erzielen.

Die im Gel dispergierten wirkstoffhaltigen Lipidpartikel haben in der Regel kugelähnliche Gestalt, sie werden daher im folgenden als "Liposphären" bezeichnet.

Eine topisch applizierbare pharmazeutische Zusammensetzung der erfindungsgemäßen Art eignet sich insbesondere für die Zubereitung von Dermatika oder von Mitteln mit antiinflammatorischer Wirkung, insbesondere Rheumamitteln; darüber hinaus von Wundheilmitteln, hypoallergenen Kosmetika, hypoallergenen Dermatika, dermalen Vitaminpräparaten, Aknetherapeutika, Antimykotika, Antibiotika, Antipsoriatika, Corticoidpräparaten und Antipruritika, weiters, insbesondere mit Ibuprofen, vorzugsweise S(+)-lbuprofen als Wirkstoff, zur Behandlung von Neurodermatitis.

Im folgenden wird die Erfindung anhand von Beispielen näher beschrieben:

### Beispiel 1:

150,o g S(+)-lbuprofen werden unter Erwärmen in 300,0 g Ricinusöl unter Ausschluß von Kristallbildung gelöst. Die erkaltete hochkonzentrierte Lösung wird anschließend in 2650 g 1%iges Polyacrylatgel (bekannt unter dem Handelsnamen "Carbopol 940 NF") in einem Labor-Salbenmischer (Fa. Haagen & Rinau) ohne Homogenisator eingearbeitet. Der pH-Wert des erhaltenen Gels beträgt 5,5. Die durchschnittliche Partikelgröße der "Liposphären" beträgt 20-30 µm.

### Beispiel 2:

7,5 kg S(+)-lbuprofen werden unter Erwärmen in 15 kg mittelkettigen Triglyceriden (bekannt unter dem Handelsnamen "Miglyol 812") unter Ausschluß von Kristallbildung gelöst. Die erkaltete hochkonzentrierte Lösung wird anschließend in 277,5 kg 1%iges Polyacrylatgel (bekannt unter dem Handelsnamen "Carbopol 980 NF") in einer Salbenanlage (Fa. Brogli) mittels Homogenisator eingearbeitet. Der pH-Wert des erhaltenen Gels beträgt 5,5. Die durchschnittliche Partikelgröße der "Liposphären" beträgt 5-10 µm.

### Beispiel 3:

20,0 g Phenylsalicylat werden in 100,0 g mittelkettigen Triglyceriden (bekannt unter dem Handelsnamen "Tegosoft CT") gelöst und die entstandene Lösung in 880,0 g 1%iges Polyacrylatgel (bekannt unter dem Handelsnamen "Carbopol 980") unter Einsatz eines Homogenisators eingearbeitet. Der pH-Wert des erhaltenen Gels beträgt 5,9. Die durchschnittliche Partikelgröße der "Liposphären" beträgt 5-10 µm.

### Beispiel 4:

10,0 g Vitamin E-acetat werden in 100,0 Neutralöl (bekannt unter dem Handelsnamen "Miglyol 812") gelöst und die entstandene Lösung mittels Homogenisator in 890,0 g 1%iges Polyacrylatgel (bekannt unter dem Handelsnamen "Carbopol 980") eingearbeitet. Der pH-Wert des erhaltenen Gels beträgt 5,8. Die durchschnittliche Partikelgröße der "Liposphären" beträgt 5-15 µm.

### Beispiel 5:

23,o g Nicotinsäurebenzylester werden in 117,0 g Mandelöl gelöst und die entstandene Lösung mittels Homogenisator in 860,0 g 1%iges Polyacrylatgel (bekannt unter dem Handelsnamen "Carbopol 980") eingearbeitet. Der pH-Wert des erhaltenen Gels beträgt 5,85. Die durchschnittliche Partikelgröße der "Liposphären" beträgt 5-15 um.

### Beispiel 6:

20,0 g Vitamin A-acetat werden in 100,0 Avocadoöl gelöst und die entstandene Lösung in 880,0 g 1%iges Polyacrylatgel (bekannt unter dem Handelsnamen "Carbopol 980") mittels Homogenisator eingearbeitet. Der pH-Wert des erhaltenen Gels beträgt 5,9. Die durchschnittliche Partikelgröße der "Liposphären" beträgt 5-15 µm.

### Beispiel 7:

5,0 g Hexetidin reinst werden in 115,0 g mittelkettigen Triglyceriden (bekannt unter dem Handelsnamen "Tegosoft CT") gelöst. 15,0 g Allantoin werden mit 35,0 g Methylcellulose (bekannt untere dem Handelsnamen "Methocel E 10 MCR Premium") und 830,0 g Wasser zu einem Hydrogel verarbeitet, wobei das Allantoin gelöst wird. Beide Phasen werden mittels Homogenisator miteinander vereinigt. Der pH-Wert des erhaltenen Gels beträgt 6,78. Die durchschnittliche Partikelgröße der "Liposphären" beträgt 15-20 µm.

### Beispiel 8:

5,0 g S(+)-lbuprofen werden unter Erwärmen in 10,0 g Ricinusöl unter Ausschluß von Kristallisation gelöst. Die erkaltete Lösung wird händisch in 85,0 g 5%iges Hydroxyethylzellulosegel (bekannt unter dem Handelsnamen "Tylose H 4000") eingearbeitet. Der pH-.Wert des erhaltenen Gels beträgt 4,22. Die durchschnittliche Partikelgröße der "Liposphären" beträgt 15 µm.

### Beispiel 9:

20,0 g Hydroxyethylrutoside werden mit 10,0 g Polyacrylat (bekannt unter dem Handelsnamen "Carbopol 940") und der entsprechenden Menge Natriumhydroxid zu 850 g Hydrogel mit pH 6,0 verarbeitet, wobei das Hydroxyethylrutosid in eine im Gel gelöste Form übergeführt wird. In 100,0 g Ricinusöl werden 50 g S(+)-lbuprofen gelöst und die Lösung mittels Homogenisator in die Gelphase eingebracht. Der pH-Wert des erhaltenen Gels beträgt 5,6. Die durchschnittliche Partikelgröße der "Liposphären" beträgt 15-25 µm.

### Beispiel 10:

1,0 g Diclofenac-Natrium werden unter leichtem Erwärmen in 10,0 g Ricinusöl gelöst. Die erkaltete Lösung wird anschließend in 89,0 g 1%iges Polyacrylatgel (bekannt unter dem Handelsnamen "Carbopol 980") eingearbeitet. der pH-Wert des erhaltenen Gels beträgt 5,6. Die durchschnittliche Partikelgröße der "Liposphären" beträgt 10-15 µm.

## Patentansprüche

1. Topisch applizierbare pharmazeutische Zusammensetzung, welche enthält:
zumindest ein flüssiges Lipid,
zumindest einen pharmazeutischen Wirkstoff, der im zumindest einem der flüssigen Lipide löslich ist und von der Haut resorbiert wird,
und ein wasserhältiges Gel, das den Hauptbestandteil der Zusammensetzung bildet, wobei zumindest ein Lipid und zumindest ein Wirkstoff in das Gel eingearbeitet sind und die Zusammensetzung im wesentlichen frei ist von Emulgatoren und festen Bestandteilen, mit Ausnahme zur Bildung des wasserhältigen Gels nötiger Geliermittel,
dadurch gekennzeichnet, daß alle Wirkstoffe in gelöster Form vorliegen, wobei jedoch zumindest ein Wirkstoff im flüssigen Lipid in gelöster Form vorliegt und daß dieses den Wirkstoff gelöst enthaltende Lipid als solches als innere Phase in das wasserhältige Gel als äußere Phase eingearbeitet ist, und die Zusammensetzung im wesentlichen frei ist von oberflächenaktiven Substanzen.

2. Zusammensetzung nach Anspruch 1, dadurch gekennzeichnet, daß sie im wesentlichen frei ist von flüchtigen organischen Lösungsmitteln und/oder im wesentlichen frei ist von flüchtigen niederen Alkanolen.

3. Zusammensetzung nach Anspruch 1 oder 2, dadurch gekennzeichnet, daß sie im wesentlichen frei ist von mit Wasser mischbaren Cosolventien, wie z.B. Propylenglycol, Glycerin oder niedermolekularen flüssigen Polyolen, z.B. Polyethylenglycol.

4. Zusammensetzung nach einem der Ansprüche 1 bis 3, dadurch gekennzeichnet, daß als flüssiges Lipid Ricinusöl und bzw. oder Mandelöl und bzw. oder Sesamöl und bzw. oder mittelkettige Triglyceride und bzw. oder Mischungen dieser Substanzen eingesetzt sind.

5. Zusammensetzung nach einem der Ansprüche 1 bis 4, dadurch gekennzeichnet, daß das Gel Hydroxyethylzellulose und bzw. oder Hydroxypropylzellulose und bzw. oder Polyacrylat enthält.

6. Zusammensetzung nach einem der Ansprüche 1 bis 5, dadurch gekennzeichnet, daß ihr Wasseranteil bis zu 90 Gew.-% (bezogen auf die Gesamtmenge) beträgt.

7. Zusammensetzung nach einem der Ansprüche 1 bis 6, dadurch gekennzeichnet, daß zumindest ein Wirkstoff im Lipid hochkonzentriert, vorzugsweise in übersättigter Lösung vorliegt und/oder daß zusätzlich zumindest ein Wirkstoff in nicht im Lipid gelöster Form im Gel vorliegt, insbesondere in in der wässerigen Gelphase gelöster Form.

8. Zusammensetzung nach einem der Ansprüche 1 bis 7, dadurch gekennzeichnet, daß als Wirkstoff lbuprofen, insbesondere S(+)-lbuprofen eingesetzt ist.

9. Zusammensetzung nach einem der Ansprüche 1 bis 8, dadurch gekennzeichnet, daß zumindest ein Wirkstoff ein nichtsteroidales Antirheumatikum ist, z.B. Diclofenac, Ketoprofen, Piroxicam, Indometacin, Flufenaminsäure, Felbinac, Hydroxyethylsalicylat, Etofenamat, Naproxen, und/oder daß zumindest ein Wirkstoff Prednisolon, Fluocortolon, Triamcinolon, Hydrocortison, Fusidinsäure, Clotrimazol, Ciclopiroxolamin, Tolnaftat, Amphotericin B, Dithranol, Vitamin A, Vitamin E, Benzoylperoxid, Hexetidin, Estradiol, Bufexamac, Polidocanol, Nicotinsäurebenzylester oder Ethylenglycolmonosalicylat ist.

10. Zusammensetzung nach einem der Ansprüche 7 bis 9, dadurch gekennzeichnet, daß zumindest ein Wirkstoff Gentamycin, Neomycin, Bacitracin, Clindamycin, Erythromycin, Aciclovir, Vidarabin, Dexpanthenol, Allantoin oder Hirudin ist.

11. Verfahren zur Herstellung einer topisch applizierbaren pharmazeutischen Zusammensetzung nach einem der Ansprüche 1 bis 10, dadurch gekennzeichnet, daß zumindest ein von der Haut resorbierbarer Wirkstoff in einem Lipid, vorzugsweise konzentriert, gelöst wird und daß sodann diese Lösung als innere Phase in ein Hydrogel als äußere Phase eingearbeitet wird, wobei das Hydrogel den Hauptbestandteil der Zusammensetzung bildet.

12. Verfahren nach Anspruch 11, dadurch gekennzeichnet, daß die Lösung des Wirkstoffes im Lipid unter Erwärmung erfolgt und daß die Lösung abgekühlt wird, bevor sie in das Gel eingearbeitet wird.

13. Verfahren nach Anspruch 11 oder 12, dadurch gekennzeichnet, daß die Lösung des Wirkstoffes im Lipid bis zur Erreichung einer übersättigten Lösung fortgesetzt wird.

14. Verfahren nach einem der Ansprüche 11 bis 13, dadurch gekennzeichnet, daß das Einarbeiten der Lösung in das Gel durch Mischen bis zur Erreichung des gewünschten Dispersionsgrades erfolgt.

15. Verwendung einer topisch applizierbaren pharmazeutischen Zusammensetzung nach einem der Ansprüche 1 bis 10
für die Zubereitung eines Dermatikums, insbesondere eines hypoallergenen Dermatikums oder
für die Zubereitung eines antibiotisch wirksamen Dermatikums oder
für die Zubereitung eines Mittels mit antiinflammatorischer Wirkung, insbesondere eines Rheumamittels, oder
für die Zubereitung eines Wundheilmittels oder
für die Zubereitung eines Mittels gegen Herpes simplex (Fieberbläschen) oder
für die Zubereitung eines Hämorrhoidenmittels oder
für die Zubereitung eines Aknetherapeutikums oder
für die Zubereitung eines Antipsoriatikums oder
für die Zubereitung eines Antimykotikums oder
für die Zubereitung eines dermalen Glucocorticoid-Präparates oder
für die Zubereitung eines Mittels zur Behandlung von Neurodermatitis.

## Claims

1. A topically applicable pharmaceutical composition, containing:
at least one liquid lipid,
at least one pharmaceutically active substance, which is soluble in at least one of the liquid lipids and is absorbed by the skin,
and a water-containing gel which forms the main constituent of the composition, wherein at least one lipid and at least one active substance are incorporated into the gel and the
composition is substantially free from emulsifiers and solid constituents, with the exception of gelling agents necessary for forming the water-containing gel,
characterised in that all active substances are present in a dissolved form, although at least one active substance is present in the liquid lipid in a dissolved form, and that this lipid containing the active substance in the dissolved form is incorporated as such as the inner phase into the water-containing gel as the outer phase, and the composition is substantially free from surfactants.

2. A composition according to Claim 1, characterised in that it is substantially free from volatile organic solvents and/or substantially free from volatile lower alkanols.

3. A composition according to Claim 1 or 2, characterised in that it is substantially free from water-miscible co-solvents, such as e.g. propylene glycol, glycerin or low-molecular liquid polyols, e.g. polyethylene glycol.

4. A composition according to one of Claims 1 to 3, characterised in that castor oil and/or almond oil and/or sesame oil and/or medium-chain triglycerides and/or mixtures of these substances are used as the liquid lipid.

5. A composition according to one of Claims 1 to 4, characterised in that the gel contains hydroxyethyl cellulose and/or hydroxypropyl cellulose and/or polyacrylate.

6. A composition according to one of Claims 1 to 5, characterised in that it contains water in a proportion of up to 90% by weight (with reference to the total quantity).

7. A composition according to one of Claims 1 to 6, characterised in that at least one active substance is present in the lipid as a high concentrate, preferably in supersaturated solution and/or that, in addition, at least one active substance is present in the gel in a form not dissolved in the lipid, in particular in a form dissolved in the aqueous gel phase.

8. A composition according to one of Claims 1 to 7, characterised in that ibuprofen, in particular S(+)-ibuprofen, is used as the active substance.

9. A composition according to one of Claims 1 to 8, characterised in that at least one active substance is a non-steroidal antirheumatic, e.g. diclofenac, ketoprofen, piroxicam, indomethacin, flufenamic acid, felbinac, hydroxyethyl salicylate, etofenamate, naproxen, and/or that at least one active substance is prednisolone, fluocortolone, triamcinolone, hydrocortisone, fusidic acid, clotrimazole, ciclopiroxolamine, tolnaftate, amphotericin B, dithranol, vitamin A, vitamin E, benzoyl peroxide, hexetidine, estradiol, bufexamac, polidocanol, nicotinic acid benzyl ester, or ethylene glycol monosalicylate.

10. A composition according to one of Claims 7 to 9, characterised in that at least one active substance is gentamycin, neomycin, bacitracin, clindamycin, erythromycin, acyclovir, vidarabine, dexpanthenol, allantoin or hirudin.

11. A method of manufacturing a topically applicable pharmaceutical composition according to one of Claims 1 to 10, characterised in that at least one active substance absorbable by the skin is dissolved in a lipid, preferably as a concentrate, and that this solution is then incorporated as an inner phase in a hydrogel as an outer phase, the hydrogel forming the main constituent of the composition.

12. A method according to Claim 11, characterised in that the active substance is dissolved in the lipid under heating, and that the solution is cooled before being incorporated in the gel.

13. A method according to Claim 11 or 12, characterised in that the dissolution of the active substance in the lipid is continued until a supersaturated solution is obtained.

14. A method according to one of Claims 11 to 13, characterised in that the solution is incorporated into the gel by mixing until the desired degree of dispersion is achieved.

15. Use of a topically applied pharmaceutical composition according to one of Claims 1 to 10
for the preparation of a dermatic agent, in particular a hypoallergenic dermatic agent or
for the prepraration of a dermatic agent with antibiotic effect or
for the preparation of a substance with anti-inflammatory action, in particular an anti-rheumatic, or
for the preparation of a substance for healing wounds or
for the preparation of a substance to control herpes simplex (cold sores) or
for the preparation of an anti-haemorrhoidal substance or
for the preparation of a substance for the treatment of acne or
for the preparation of an antipsioratic or
for the preparation of an antimycotic or
for the preparation of a dermal glucocorticoid or
for the preparation of a substance for the treatment of neurodermatitis.

## Revendications

1. Composition pharmaceutique d'application topique comprenant :
au moins un lipide sous forme liquide,
au moins un principe actif pharmaceutique soluble dans au moins un des lipides liquides et résorbé par la peau,
et un gel hydraté formant l'élément principal de la composition, au moins un lipide et au moins un principe actif étant incorporés dans le gel, et la composition étant pour l'essentiel exempte d'émulsifiants et de composants solides, à l'exception du gélifiant nécessaire à la formation du gel hydraté,
caractérisée en ce que tous les principes actifs sont présents sous forme solubilisée, un principe actif au moins étant présent sous forme solubilisée dans le lipide liquide, et ce lipide contenant le principe actif solubilisé étant incorporé en tant que phase interne dans le gel hydraté en tant que phase externe, et la composition étant pour l'essentiel exempte de substances tensioactives.

2. Composition selon la revendication 1, caractérisée en ce qu'elle est pour l'essentiel exempte de solvants organiques volatiles et/ou pour l'essentiel exempte d'alcools inférieurs volatiles.

3. Composition selon la revendication 1 ou 2, caractérisée en ce qu'elle est pour l'essentiel exempte de co-solvants miscibles à l'eau, comme par exemple le propylène-glycol, la glycérine ou les polyols liquides de faible poids moléculaire, par exemple le polyéthylène-glycol.

4. Composition selon l'une des revendications 1 à 3, caractérisée en ce qu'on utilise, comme lipide liquide, de l'huile de ricin et/ou de l'huile d'amande et/ou de l'huile de sésame et/ou des triglycérides à chaîne moyenne et/ou des mélanges de ces substances.

5. Composition selon l'une des revendications 1 à 4, caractérisée en ce que le gel contient de l'éthyl-cellulose hydroxylée et/ou de la propylcellulose hydroxylée et/ou du polyacrylate.

6. Composition selon l'une des revendications 1 à 5, caractérisée en ce-que sa teneur en eau va jusqu'à 90 % en poids (rapporté au volume total).

7. Composition selon l'une des revendications 1 à 6, caractérisée en ce qu'au moins un principe actif est présent, dans le lipide, sous forme hautement concentrée, de préférence en solution sursaturée et/ou qu'au moins un principe actif est présent dans le gel sous une forme non solubilisée dans le lipide, notamment sous forme solubilisée dans la phase aqueuse du gel.

8. Composition selon l'une des revendications 1 à 7, caractérisée en ce qu'on utilise, comme principe actif, de l'ibuprofène, notamment du S(+)-ibuprofène.

9. Composition selon l'une des revendications 1 à 8, caractérisée en ce qu'au moins un principe actif est un antirhumatismal non-stéroïdien, par exemple diclofenac, kétoprofène, piroxicame, indométacine, acide flufénamique, felbinac, hydroxyéthylsalicylate, étofénamate, naproxène et/ou qu'au moins un principe actif est de la prednisolone, fluocortolone, triamcinolone, hydrocortisone, acide fusidinique, clotrimazole, ciclopiroxolamine, tolnaftate, amphotéricine B, dithranol, vitamine A, vitamine E, benzoylpéroxide, hexétidine, estradiol, bufexamac, polydocanol, ester benzylique de l'acide nicotinique ou éthylèneglycolmonosalicylate.

10. Composition selon l'une des revendications 7 à 9, caractérisée en ce qu'au moins un principe actif est de la gentamycine, néomycine, bacitracine, clindamycine, érythromycine, aciclovir, vidarabine, dexphanténol, allantoïne ou hirudine.

11. Procédé de fabrication d'une composition pharmaceutique d'application topique selon l'une des revendications 1 à 10, caractérisée en ce qu'au moins un principe actif résorbable par la peau est solubilisé dans un lipide, de préférence de façon concentrée, et que cette solution est alors incorporée, en tant que phase interne, dans un hydrogel en tant que phase externe, l'hydrogel étant le composant majeur de la composition.

12. Procédé selon la revendication 11 caractérisé en ce que la dissolution du principe actif dans le lipide se fait sous l'effet de la chaleur et que la solution est refroidie avant d'être incorporée dans le gel.

13. Procédé selon la revendication 11 ou 12 caractérisé en ce que la dissolution du principe actif dans le lipide est poursuivie jusqu'à l'obtention d'une solution sursaturée.

14. Procédé selon l'une des revendications 11 à 13 caractérisé en ce que l'incorporation de la solution dans le gel se fait par mélange jusqu'à l'obtention du taux de dispersion souhaité.

15. Utilisation d'une composition pharmaceutique d'application topique selon l'une des revendications 1 à 10
pour la préparation d'un médicament dermique, notamment d'un médicament dermique hypoallergène ou
pour la préparation d'un médicament dermique à effet antibiotique ou
pour la préparation d'un produit à effet anti-inflammatoire, notamment d'un antirhumatismal ou
pour la préparation d'un produit cicatrisant ou
pour la préparation d'un produit contre l'herpes simplex (boutons de fièvre) ou
pour la préparation d'un produit antihémorrhoïdal ou
pour la préparation d'un produit de traitement de l'acnée ou
pour la préparation d'un antipsoriasique ou
pour la préparation d'un antimycotique ou
pour la préparation d'un glucocorticoïde dermique ou
pour la préparation d'un produit pour le traitement de la neurodermite.
